(19) Europäisches Patentamt
European Patent Office
Office européen des brevets

(11) EP 2 565 197 A1

# (12) EUROPEAN PATENT APPLICATION

(43) Date of publication:
**06.03.2013 Bulletin 2013/10**

(51) Int Cl.:
***C07D 493/22*** *(2006.01)*

(21) Application number: **11007018.2**

(22) Date of filing: **29.08.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Max-Planck-Gesellschaft zur Förderung der Wissenschaften e.V.**
**80539 München (DE)**

(72) Inventors:
- **Seeberger, Peter H., Prof. Dr.**
  **14532 Kleinmachnow (DE)**
- **Lévesque, Francois, Dr.**
  **12203 Berlin (DE)**
- **Kopetzki, Daniel, Dr.**
  **10623 Berlin (DE)**

(74) Representative: **Arth, Hans-Lothar**
**ABK Patent Attorneys**
**Jasminweg 9**
**14052 Berlin (DE)**

## (54) Method and device for the synthesis of artemisinin

(57) The present invention is directed to a method for producing artemisinin having the formula from dihydroartemisinic acid in a continuous flow reactor using singlet oxygen.

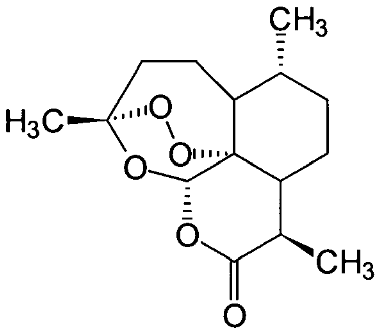

EP 2 565 197 A1

Figure 4

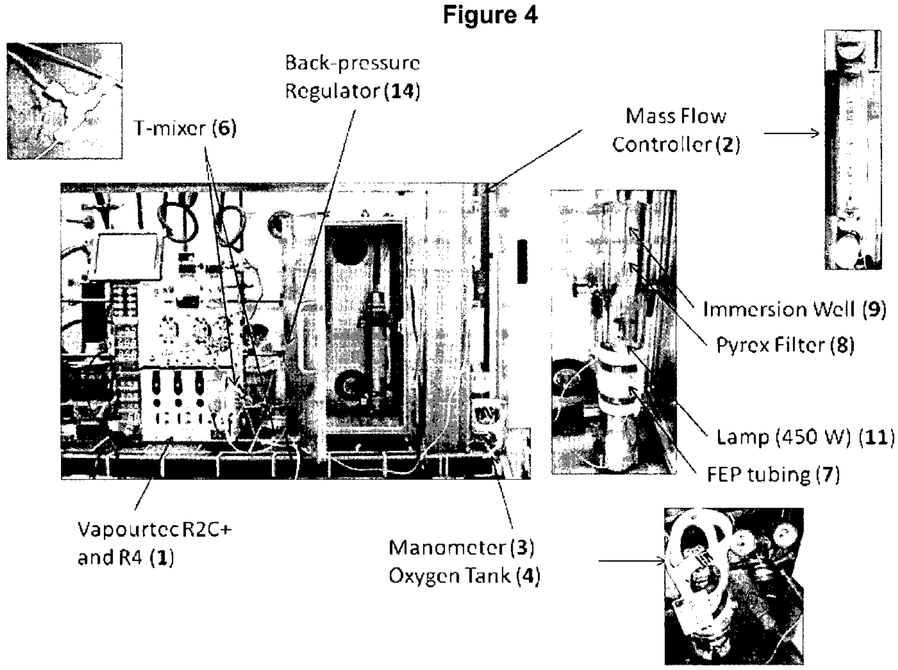
Back-pressure Regulator (14)
T-mixer (6)
Mass Flow Controller (2)
Immersion Well (9)
Pyrex Filter (8)
Lamp (450 W) (11)
FEP tubing (7)
Vapourtec R2C+ and R4 (1)
Manometer (3) Oxygen Tank (4)

## Description

Malaria, caused by the protozoan parasite *Plasmodium falciparum,* remains a major global health problem that kills almost one million people each year. Artemisinin is currently the most effective treatment against multi-drug resistant *Plasmodium* species and artemisinin combination treatments (ACTs) are now first-line drugs (World Malaria Report 2010, WHO Geneva, 2010). Access to the sesquiterpene endoperoxide molecule relies on extraction from the plant *Artimisia annua* (sweet wormwood) that has been cultivated in many countries for that purpose (White, N. J. Science, 2008, 320, 330-334). The natural synthesis of artemisinin starts with isopentenyl pyrophosphate (IPP) and its isomer dimethylallyl pyrophosphate to dihydroartemisinic acid and involves as intermediate product amorpha-4,11-diene (Brown, G. D. Molecules, 2010, 15, 7603-7698). Total synthesis of artemisinin is too laborious to be considered viable for supplying the highly cost sensitive market. Artemisinic acid, a much less complex molecular precursor can be extracted from the same plant in higher yields and can be produced in engineered *Saccharomyces cerevisiae* (Ro, D.-K., et al., Nature, 2006, 440, 940-943). Therefore, artemisinic acid is a good starting point for the synthesis of the drug substance. Still, the conversion of artemisinic acid to artemisinin has proven a formidable challenge for chemists since a high-yielding and scalable low cost process for the construction of this highly complex molecule is needed.

WO 2009/088404 A1 discloses a synthesis for artemisinin starting from dihydroartemisinic acid

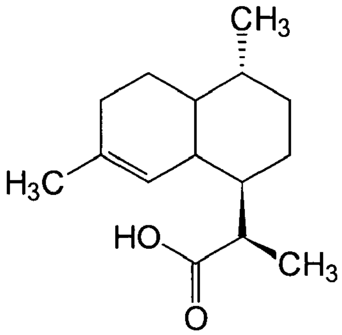

which is in a first step converted to a dihydroartemisinic acid ester of the following formula

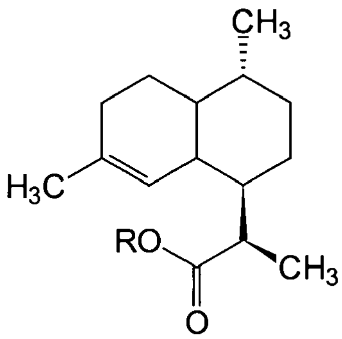

wherein R is an alkyl, alkenyl, alkynyl, aryl, cycloalkyl, alkaryl or aralkyl group. The dihydroartemisinic acid ester is than reacted with a peroxidizing agent in order to obtain the dihydroartemisinic acid ester peroxide of the following formula

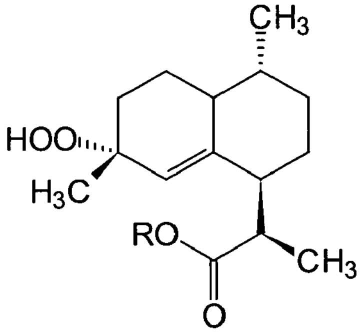

which is than converted to artemisinin by the reaction with oxygen. This four step synthesis is quite long and requires optimization especially for large scale synthesis in order to shorten this synthesis route, to reduce labor costs, purification efforts and to increase yield.

Thus there is a need to provide a more efficient synthesis of artemisinin.

Molecular oxygen is an attractive reagent due to its availability, low cost and negligible environmental impact. Singlet oxygen ($^1O_2$) is formed via dye-sensitized photoexcitation of triplet oxygen ($^3O_{2)}$ and facilitates heteroatom oxidations, ene reactions, as well as [4+2] and [2+2] cycloadditions ( a) Schweitzer, C.; Schmidt, R. Chem. Rev., 2003, 103, 1685-1758. b) Hoffmann, N. Chem. Rev. 2008, 108, 1052-1103.). Consequently, $^1O_2$ has been used for the synthesis of natural products and fragrances. Widespread use of $^1O_2$ in conventional batch systems has been prevented by the need for specialized equipment to produce the reagent and technical challenges associated with scaling-up photochemical reactions and the low rate of mass transfer of oxygen gas.

Continuous flow reactors allow for easy scale-up as no change in reactor size is required, provide a large surface-to-volume ratio that ensures efficient irradiation and enable precise control over the reaction time to minimize unwanted side reactions due to secondary photochemical reactions. In addition, continuous flow reactors improve safety as reactive intermediates are quenched or further transformed immediately after production. Photochemical flow reactors have been explored for the generation and use of $^1O_2$. Although complete conversion was achieved in a short residence time, the process suffered from very low productivity, rendering the system inapplicable to use on industrial scale. Alternatively, this problem of low $O_2$(g) mass transfer has been tackled by using supercritical carbon dioxide as a solvent, though this mandates a highly specialized reaction set-up.

Efficient oxidations are dependent on the solution concentration of $^1O_2$, which in turn is proportional to the solution concentration of $^3O_2$. Therefore, the productivity of the oxidation depends on the rate of mass transfer (d[$^3O_2$ (sol)]/dt) of $^3O_2$(g) into the solution. Based on Fick's Law (Equation {1}) the rate of mass transfer is determined by the liquid film transfer coefficient ($K_L$), the specific surface area of the solution (a) and the oxygen deficit within the solution ([$^3O_{2(sol)}$]sat-[$^3O_{2(sol)}$]).

$$d[^3O_{2(sol)}]/dt = K_L a([^3O_{2(sol)}]sat-[^3O_{2(sol)}]) \qquad \{1\}$$

When biphasic gas-liquid reactions are conducted at high flow rates, the specific surface areas in continuous flow reactors (up to 25300 $m^2/m^3$) can greatly exceed those attained in conventional batch reactors (up to 2000 $m^2/m^3$) due to flow pattern effects. To date, synthetic organic chemists have not taken full advantage of variations in the flow patterns of biphasic reactions.

Objective of the present invention is to provide a more efficient alternative synthesis of artemisinin.

The objective of the present invention is solved by the teaching of the independent claims. Further advantageous features, aspects and details of the invention are evident from the dependent claims, the description, the figures, and the examples of the present application.

It was found that artemisinin can be prepared in a one continuous flow process from dihydroartemisinic acid (2) by the use of singlet oxygen. A simple continuous flow system renders reactions involving singlet oxygen ($^1O_2$) practical for large scale synthetic synthesis of artemisinin. Efficient mass transfer and sufficient irradiation enable the straightforward production and use of $^1O_2$ as a reagent in one continuous flow process for synthesis of artemisinin (6) starting from dihydroartemisinic acid (2). Dihydroartemisinic acid is commercially available from Honsea Sunshine Biotech Co., Ltd. or can be produced by a modified yeast (Zhang, Y. et al. J. Biol. Chem. 2008, 31, 21501-21508).

Thus the present invention is directed to a method for producing artemisinin (6) from dihydroartemisinic acid

(2) comprising or consisting of the following steps:

A) providing dihydroartemisinic acid (2) represented by the following formula

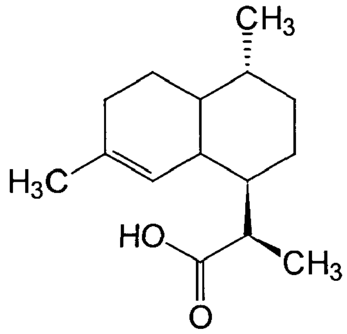

B) performing in a continuous flow reactor the following reactions

i) photooxidation of dihydroartemisinic acid (2) with singlet oxygen,
ii) followed by an acid mediated cleavage and
iii) subsequent oxidation with triplet oxygen in order to obtain artemisinin (6) of the following formula:

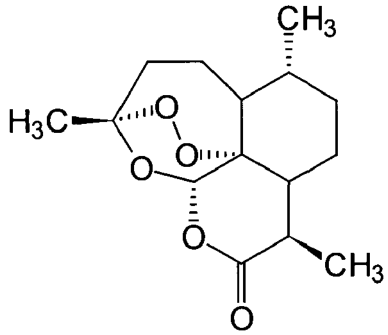

In case a chemical synthesis of dihydroartemisinic acid (2) is desired, dihydroartemisinic acid (2) can be prepared by reducing artemisinic acid (1). Thus, dihydroartemisinic acid (2) can be obtained by reducing artemisinic acid (1) of the following formula

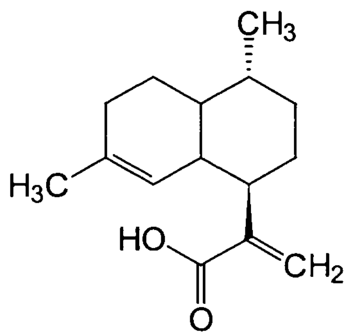

to dihydroartemisinic acid (2).

Thus the present invention is also directed to a method for producing artemisinin (6) from artemisinic acid (1) comprising the following steps:

A) providing artemisinic acid (1) represented by the following formula

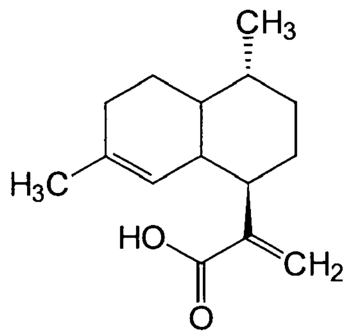

B) reducing artemisinic acid (1) to dihydroartemisinic acid (2) of the following formula

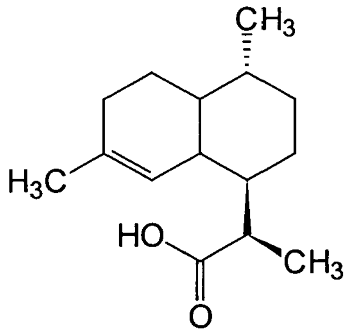

C) performing in a continuous flow reactor the following reactions

i) photooxidation of dihydroartemisinic acid (2) with singlet oxygen,
ii) followed by an acid mediated cleavage and
iii) subsequent oxidation with triplet oxygen
in order to obtain artemisinin (6) of the following formula:

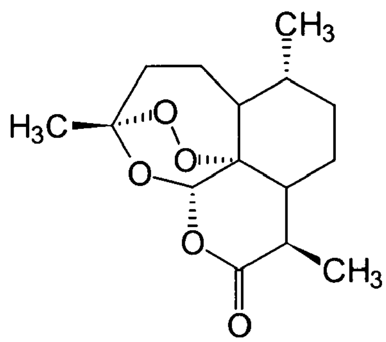

The starting material artemisinic acid (1) which is also known as arteannuic acid and which has the chemical name 2-[(1 R,4R,4aS,8aR)-4,7-dimethyl-1,2,3,4,4a,5,6,8a-octahydronaphthalen-1-yl]prop-2-enoic acid can be obtained synthetically, by recombinant methods or can be isolated from the plant *Artemisia annua.* Since there is much more artemisinic acid contained in the plant *Artemisia annua* than artemisinin, an efficient method to convert artemisinic acid (1) into artemisinin (6) is desired.

Artemisinin (6) belongs to the group of the sesquiterpenes and is isolated from the leaves and blossoms of the plant *Artemisia annua.* Artemisinin (6) has an uncommon trioxane ring structure and a peroxide brigde and has been used for a long time as anti-malaria drug.

Artemisinic acid (1) and dihydroartemisinic acid (2), both bicyclic molecules, lack much of the complexity that imparts the biological activity to artemisinin (6), a sesquiterpene endoperoxide with a dense array of functional groups. Access to large quantities of artemisinic acid via engineered yeast (Ro, D.K. et al. Nature, 2006, 440, 940-943), rendered artemisinic acid (1) an attractive starting material for chemical semi-synthesis of artemisinin (6). The conversion of artemisinic acid (1) to artemisinin (6) involves several challenging steps that were examined in the context several synthetic endeavors ( a) Roth, R. J.; Roth, N. A. United States Patent 4,992,561 1991 b) Reiling, K. K.; Renninger, N. S.; McPhee, D. J.; Fisher, K. J.; Ockey, D. A. International Patent WO 2006/128126 A1 2006**.** c) Roth, R. J.; Acton, N. J. Chem. Edu. 1991, 68, 612-613. d) Roth, R. J.; Acton, N. A J. Nat. Prod. 1989, 52, 1183-1185. e) Constantino, M. G.; Beltrame Jr. M.; da Silva, G. V. J. Syn. Comm. 1996, 26, 321-329.). Current solutions to this problem are technically too complex for scale-up while meeting stringent cost targets. Photochemical transformations have the advantage that light is a relatively inexpensive and mild reagent, but were not used often for drug synthesis since they are hard to scale up. The distance light can penetrate through solutions is limited due to absorption and moving to larger reactions vessels greatly diminishes conversion and yield.

In order to overcome these drawbacks of photochemical reactions, the inventors designed a continuous flow set-up which in contrast to batch processes, allows for the production of large quantities of material by simply extending the run time rather than changing to larger reaction vessels. Excellent control over reaction parameters such as reaction time, temperature and mixing are hallmarks of flow chemistry. Photochemical transformations greatly benefit from the flow regime as the penetration depth of the light remains the same during scale-up due to the size of the tubing that serves as reaction vessel. Simple setups consist of lamps, surrounded by cooling devices that are wrapped with tubing to carry out the reactions. Intending to utilize continuous flow chemistry as a means to scale-up photochemical transformations the inventors examined the transformation of artemisinic acid (1) or dihydroartemisinic acid (2) to artemisinin (6) mindful of the necessity to create a simple, scalable and inexpensive process.

Artemisinic acid (1) can be efficiently reduced to dihydroartemisinic acid (2) using batch methods such as diimide on large scale (WO 2011/030223 A2). The three-step reaction sequence to convert dihydroartemisinic acid (2) to artemisinin (6) involving photochemically induced oxidation with singlet oxygen, acid-mediated Hock cleavage ( a) Lange, J.-P.; Breed, A. J. M. Catal. Comm. 2002, 3, 25-28. b) Olah, G. A.; Parker, D. G.; Yoneda, N. Angew. Chem. Int. Ed. Engl. 1978, 17, 909-931). and oxidation with triplet oxygen (Chen, B.-C.; Zhou, P.; Davis, F. A.; Ciganek, E. Organic Reactions 2004, 64, 1— 356). poses synthetic challenges which were addressed by the present invention and which were solved in a way that all three steps starting from dihydroartemisinic acid (2) could be combined in a continuous flow system which can be easily controlled, easily scaled up, obtained good yields and does not required the isolation and purification of any intermediates.

The photooxidation of dihydroartemisinic acid (2) results in the intermediate products (3), (4) and (5) as described in example 1. The main intermediate product is the hydroperoxide (3) which is obtained in at least 75% yield, preferably in at least 80% yield and more preferably in at least 84% yield. In order to perform the following reactions it is not required to purify the obtained hydroperoxide (3) or to remove the intermediate products (4) and (5) as shown in example 1.

The hydroperoxide (3) of the following formula

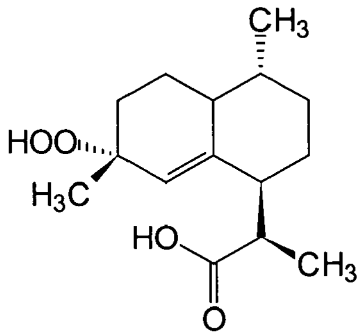

is formed as photooxidation product from the reaction of dihydroartemisinic acid (2) with singlet oxygen in the photochemical reactor and more precisely in the solution of dihydroartemisinic acid (2) and oxygen running through the tubing **7** which is wrapped around the filter **8,** the light source **11** and the immersion well **9** of the photochemical reactor while this solution is irradiated by the light source **11.** The outflow from the tubing 7 can be processed in three different ways,

namely in a batch process, or in a separate continuous flow process, or in a continued continuous flow process.

The hydroperoxide (3) contained in the outflow of the tubing 7 has to undergo an acid mediated cleavage of the hydroperoxide bond followed by subsequent oxidation preferably with oxygen (more specific with triplet oxygen) in order to obtain artemisinin (6).

Thus as outlined above, the hydroperoxide (3) is produced in the photochemical reactor of the present invention in a continuous flow process, while the next reactions can be performed by continuing said continuous flow process, starting a new continuous flow process or switching to a batch process.

In case of switching to a batch process, it is still not necessary to purify the hydroperoxide (3) or to remove the intermediate products (4) and (5). The obtained hydroperoxide (3) is simply collected in a batch reactor like a flask, is flushed with oxygen or an oxygen containing gas such as air, and treated with an acid such as TFA in order to obtain artemisinin (6). This method combining continuous flow through the photochemical reactor with subsequent batch process is described in example 2.

The second possibility of starting a new continuous flow process includes collecting the reaction solution coming out of the tubing which contains the hydroperoxide (3) and the intermediate products (4) and (5). No purification step or removal of the intermediate products (4) and (5) is required in order to perform the next reactions. The collected reaction solution may be concentrated or diluted and is fed into a second continuous flow reactor together with oxygen or an oxygen containing gas such as air and an acid such as TFA in order to produce artemisinin (6), while this solution is running through that second continuous flow reactor. This method combining continuous flow through the photochemical reactor with subsequent continuous flow through the second continuous flow reactor is described in example 3. This method might be preferred in a case where more material could be processed through the second continuous flow reactor than through the photochemical reactor so that probably the reaction solutions of two or more photochemical reactors are collected and then processed through one single second continuous flow reactor.

The most preferred method is to continue the continuous flow process which is most preferred if photochemical reactor and subsequent continuous flow reactor have a similar capacity. In that method the reaction solution coming out of the photochemical reactor is directly forwarded to and/or fed into the subsequent continuous flow reactor without first collecting said reaction solution. The acid is preferably added to the reaction solution coming out of the photochemical reactor and oxygen or an oxygen containing gas such as air is also fed into this reaction solution so that the hydroperoxide (3) in the reaction solution fed into the subsequent continuous flow reactor is cleaved by the acid and oxidized by the triplet oxygen to the final product artemisinin (6). This most preferred method using one single continuous flow process from the production of artemisinin (6) from dihydroartemisinic acid (2) is described in example 4.

The photooxidation of dihydroartemisinic acid (2) was explored in a home-built continuous flow reactor as shown in Figures 1 and 4, comprising a photochemical reactor consisting of fluorinated ethylene propylene (FEP) tubing wrapped around a Schenk photochemical reactor containing a 450 W medium pressure mercury lamp that was cooled to 25°C. Preferred are photochemical reactors having a volume between 10 ml and 30 ml. A solution of dihydroartemisinic acid (2) in an organic solvent was added *via* a HPLC pump and oxygen was delivered via a mass flow controller connected to a gas cylinder. The solution of (2) and the oxygen gas were mixed using a ethylene tetrafluoroethylene (ETFE) T-mixer. Tetraphenylporphyrin (TPP) was used as photosensitizer due to its high quantum yield and high stability against photo-bleaching. Alternatively, rose Bengal (R.B.) and methylene blue (M.B.) can also be used as photosensitizer. Any other photosensitizer capable of promoting the generation of singlet oxygen could be used, for example: 5,10,15,20-tetrakis(pentafluorophenyl)porphyrin, tris(2,2'-bipyridyl)ruthenium(II), pheophytin a, pheophorbide a, 2,3,7,8-dibenzopyrene-1,6-quinone and metal-phthalocyanines.

FEP or fluorinated ethylene propylene refers to a copolymer of hexafluoropropylene and tetrafluoroethylene. Other suitable fluorinated polymers are PTFE (polytetrafluoroethylene) and fluorinated ethylene propylene copolymers from DuPont sold under the brandname Teflon® FEP.

Most preferentially, the inventive method is carried out at a temperature of 25°C. In general, a preferred temperature range is between -10 and 60°C, more preferred between 20 and 40°C.

Since oxygen gas can result in severe fires a non-flammable, typically halogenated solvent is required. On large scale, halogenated solvents other than dichloromethane are considered too toxic so that dichloromethane is the preferred solvent for large scale production of artemisinin (6). Under preferred conditions, 1.50 mmol of intermediate (3) were produced per minute when a 10 ml to 60 ml reactor and preferably a 20 mL reactor was fed with 2.5 mL/min of the solution of dihydroartemisinic acid (2) in dichloromethane and oxygen was fed at a flow rate of 5 mL/min. At 91 % conversion and a yield of 75% for this step this process was better or at least as good as any process ever described for this transformation.

Protonation of the peroxide (3) is required to induce cleavage and rearrangement of the cyclic structure. The intermediate product evolved from the protonation of the endoperoxide oxygen atom of compound (3) undergoes undesired reactions which in the state of the art have been suppressed by the conversion of the carboxylic acid into an ester or a mixed anhydride. Such a transformation requires additional steps and the use of the carboxylic acid reduces considerably the formation of the 6-membered lactone product. The inventive method disclosed herein does not require

the protection of the carboxylic acid group as an ester or mixed anhydride.

Different Brønsted and Lewis acids were tested in various solvents to find the most efficient path for converting the peroxide obtained after the singlet oxygen reaction into the intermediate which is reacted with triplet oxygen to obtain artemisinin (6). The following Brønsted and Lewis acids were tested: camphorsulfonic acid, copper(II) trifluoromethanesulfonate, DOWEX®, *p*-toluenesulfonic acid and trifluoroacetic acid. Other suitable Brønsted or Lewis acids having similar pKa could be used. There are no special requirements for the acid. Trifluoroacetic acid (TFA) performed so far best as acid to induce the Hock cleavage.

It is essential to point out that a Brønsted acid or a Lewis acid needs to be added to perform the Hock cleavage. This addition could be done directly at the beginning or just after the completion of the reaction with singlet oxygen. The addition of the acid after the photooxidation reaction is preferred so that preferably the acid is added to the reaction solution leaving the photochemical reactor. Treatment of the crude tertiary allylic hydroperoxide (3) obtained from the photochemical reactor with 0.5 eq. of TFA at 0°C while bubbling oxygen (pure oxygen or air) gave, after purification by chromatography, arteminisin (6) in 50% yield.

In an effort to minimize manipulations of the reaction mixture and move to a fully continuous process, the Hock cleavage step with the action of acid and the addition of oxygen were performed in one continuous flow set-up. Best results were obtained in a, for instance, 42 mL reactor at a flow rate of, for instance, 2.5 mL/min of the crude tertiary allylic hydroperoxide (3) in dichloromethane, a flow rate of 5.0 mL/min of oxygen and a flow rate of 0.5 mL/min of TFA in dichloromethane. The first portion of the reactor (32 mL) was preferably maintained at room temperature and the last portion (10 mL) was preferably heated at 60°C to push the reaction to completion. The ensuing triplet oxygen oxidation produced desired artemisinin (6) and five member lactone byproduct in a ratio of 5.3:1.0 in favor of (6). Following purification by chromatography yielded 46% of (6) in this sequential continuous flow multistep reaction from dihydroartesiminic acid (2).

Moreover it was found that solvents with a lower dipole moment resulted in better selectivity so that the acid and especially TFA was dissolved in toluene, benzene, cyclohexane, xylene or dioxane.

The final synthetic transformation of the intermediate compound obtained after acid cleavage to artemisinin (6) requires an oxidation with triplet oxygen that is followed by a cascade of reactions that produce two rings, including the peroxide framework of artemisinin (6). According to the present invention, the Hock cleavage step with the action of acid and the addition of oxygen were performed in one continuous flow set-up as shown in Fig. 2.

After a three step process for the conversion of dihydroartemisinic acid (2) to artemisinin (6) had been developed whereby each step was performed in continuous flow and the last two steps had already been done without work-up in one flow set-up, the key question was whether one fully integrated continuous flow synthesis could be developed. A fully contained, continuous process that receives as input dihydroartemisinic acid (2), oxygen gas, photosensitizer, and an acid such as TFA at the appropriate time while allowing for a photochemical step as well as transformations without light to produce continuously artemisinin (6) is extremely attractive. For this purpose, a commercially available continuous flow system including pumping HPLC pumps was combined with the home-built photochemical flow set-up as well as further pumps and mixers (Fig. 2).

The present invention also refers to a device in which the inventive process of producing artemisinin takes place. In particular, the invention refers to a photochemical reactor for the production of artemisinin (6) from dihydroartemisinic acid (2) comprising or consisting of

- a light source **11,**
- an immersion well 9 surrounding the light source **11,**
- a filter **8** surrounding the light source **11** and
- multiple loops of a tubing **7** wrapped tightly around the filter **8,** the tubing **7** having an inlet for a mixture of dihydroartemisinic acid and oxygen on its one end and an outlet for the reacted products on the opposite end.

The immersion well **9** surrounding the light source **11** preferably in a cylinder-like shape and the filter **8** surrounding also the light source **11** preferably in a cylinder-like shape while the filter **8** could be inside the immersion well **9** or the immersion well **9** could be inside the filter **8.** If the filter **8** is inside the immersion well **9** surrounding the light source **11,** the tubing is directly wrapped around the immersion well 9 and indirectly also wrapped around the filter **8** and the light source **11.** If the immersion well **9** is inside the filter **8** surrounding the light source **11,** the tubing is directly wrapped around the filter **8** and indirectly also wrapped around the immersion well **9** and the light source **11.** However it is preferred that the light source **11** is directly surrounded by the immersion well 9 and the immersion well 9 is again surrounded by the filter **8** and the filter **8** is again surrounded by the tubing **7.**

Tightly wrapped, as used herein, means that the tubing is wrapped around the filter in such a way that it keeps tight to the underlying filter and is not liable to slide down or to be shifted involuntarily or by applying flow pressure and/or temperature to the device. On the other hand it isn't wrapped that tightly that the tubing material gets overstretched and could be easily damaged by physicochemical stress or use. The main difference between the photochemical reactor

used within the present invention and any common photochemical reactor such as the Scheck photochemical reactor is that the reactor vessel which usually surrounds the immersion well is replaced by the tubing which is wrapped around the filter and the immersion well.

A Pyrex filter is preferred as filter. Preferably, it has an inner diameter of 4.85 cm and a wall thickness of 0.28 cm. The diameter of the filter is related to the diameter of the immersion well. The filter could also be placed inside the immersion well, just around the lamp. In that case the diameter of the filter is smaller but the thickness is the same.

For the tubing of the photoreactor FEP (fluorinated ethylene polymer) is preferred as material. A preferred FEP tubing is from IDEX Health & Science, FEP 1520, natural color, outside diameter (OD) 1/16 in and inside diameter (ID) 0.030 in). In general the internal diameter (ID) can be from 0.003 in to 0.120 in. FEP tubing having the internal diameter of 0.003 in, 0.004 in, 0.008 in, 0.010 in, 0.020 in, 0.030 in, 0.062 in, 0.125 in, 0.156 in, 0.250 in, 0.5 mm, 1 mm, 2 mm and 3 mm are easily commercially available. In general the outer diameter (OD) can be from 1/32 in to 4 mm. FEP tubing having the outer diameter of 1/32 in, 1/16 in, 1/8 in, 3/16 in, 1/4 in, 5/16 in, 1 mm, 2 mm, 3 mm and 4 mm are easily commercially available. Alternatively, fluorinated or perfluorinated alkylene polymer or fluorinated ethylene propylene can be used FEP was preferably selected for the tubing due to its high transmittance and stability in the UV-vis light range, its flexibility and its high chemical resistance. Any polymer having these properties could be used instead of FEP. The immersion well is preferably made of quartz. Since a pyrex filter is used, also an immersion well made of pyrex (borosilicate) could be used.

The photochemical reactor may further comprise a thermostat **10** for cooling the immersion well **9.** Thus this photochemical reactor comprises or consists of:

- a light source **11,**
- an immersion well 9 surrounding the light source **11,**
- a thermostat **10** for cooling the immersion well **9**,
- a filter **8** surrounding the light source **11** and
- multiple loops of a tubing **7** wrapped tightly around the filter **8,** the tubing **7** having an inlet for a mixture of dihydroartemisinic acid and oxygen on its one end and an outlet for the reacted products on the opposite end.

The light source is preferably a Hg lamp and more preferably a medium pressure Hg lamp (such as Ace Glass, UV 450 immersion lamp, 5 in arc, radial lead, 7825-34). However the use of mercury lamps is not mandatory, also LEDs, fluorescent lamps or halogen lamps could be used.

In a preferred embodiment a 450 W Hg lamp is used. In general the power range can be from 100 W to 1200 W. Lamps having the powers of 100 W, 200 W, 450 W, 500 W and 1200 W are easily commercially available.

The light source needs an adapted power source **12.** For such an Ace Glass lamp an Ace Glass, 7830 power supply is preferred. However, any power source supplying this power could be used.

Optionally, the inventive photoreactor may further comprise a thermostat for cooling the immersion well.

The thermostat is for example a Huber, Unistat 360. Any cooling system able to cool down the lamp at the desired temperature could be used. For example another photochemical reactor uses a Julabo FL601. Also, one cooling system could be used for more than one photochemical reactor.

Furthermore the present invention refers as well to a flow reactor or a continuous flow reactor for the production of artemisinin (6) from dihydroartemisinic acid (2) comprising or consisting of

- the photochemical reactor described before,
- a first mixer **6a**, connected to the inlet of the tubing **7** of the photochemical reactor,
- a feed **F1** for a solution of dihydroartemisinic acid,
- a first pump **1a** for pumping the solution of dihydroartemisinic acid to the first mixer **6a,**
- a check-valve **5a** between the first pump **1a** and the first mixer **6a,**
- an oxygen tank 4 with a manometer **3,**
- a mass flow controller **2** disposed between the oxygen tank **4** and the first mixer **6a** for controlling the oxygen flow rate,
- a check valve **5b** between the mass flow controller **2** and the first mixer **6a,**
- a feed **F2** for an acidic solution,
- a second mixer **6b** connected to the outlet of the tubing **7** of the photochemical reactor and to the feed **F2** of the acidic solution,
- a second pump **1 b** for pumping the acidic solution to the second mixer **6b,**
- at least one reactor **15** for producing or completing the synthsis of artemisinin, connected downstream to the second mixer **6b,** and
- a collection flask **18** for collecting the artemisinin-containing solution from the at least one reactor **15.**

It is evident to a skilled person that the continuous flow reactor of the present invention also may comprise, for

instance, tubing for connecting said parts or fastener and clamps for fixating the single components of the continuous flow reactor. However these parts are not essential to the invention and thus are not explicitly mentioned in the patent claims.

The term "continuous flow reactor" as used herein does not mean that necessarily the complete reaction process starting from dihydroartemisinic acid (2) to produce artemisinin (6) is carried out in a continuous flow. This is one preferred possibility but not the only possibility. Moreover the term "continuous flow reactor" indicates that the inventive procedure how to perform the singlet oxygen oxidation in the photochemical reactor is carried out in a continuous process. The subsequent acid cleavage and triplet oxidation step can be carried out in a continued continuous flow process, a second continuous flow process or a batch process.

In an alternative embodiment the acidic solution flows from its feed **F2** not to the second mixer **6b** but to the first mixer **6a** and the second pump **1b** is not arranged between the feed **F2** for the acidic solution and the second mixer **6b** but between the feed **F2** for the acidic solution and the first mixer **6a.**

It is preferred that the pumps are HPLC pumps. More preferred are models such as a Vapourtec R2C+ unit. Any HPLC pumps or pumps for continuous flow systems can be used such as Syrris pumps. The only thing that needs to be mentioned is that an acid resistant pump is required in order to pump the solution of the acid such as the TFA solution.

The mass flow controller is preferably from Influx, SVIB5-AI05. It allows the control of the flow rate from 5-90 $cm^3$/min. Any mass flow controller allowing to control this range of flow rates can be used.

The oxygen tank can be for example an Air Liquide, $O_2$ (99.995% pure). However the purity of the oxygen is not important. Also air instead of 99.9% pure oxygen worked well.

The check-valves are preferably from IDEX Health and Science, inline check-valve CV-3010. The use of check-valve is preferred but not essential. Any check-valve can be used. The check-valve was used in order to prevent the solvent to go into the mass flow controller.

For both mixers ETFE T-mixers are preferred. Particularly preferred is a IDEX Health and Science, P-632. The ETFE T-mixers are preferred, because the material has high corrosion resistance and strength over a wide temperature range. Other polymers having similar properties could be used. The T-mixers could also be changed for mixer that have other shape, for example Y-mixer.

The tubing of the inventive device apart of the photoreactor is preferably of FEP. This tubing was selected for its high transmittance and stability in the UV-vis light range, its flexibility and its high chemical resistance. Any polymer having these properties could be used instead of FEP.

For the cleavage and triplet oxygen oxidation at least one PTFE reactor is preferred. Preferably two reactors **(15, 16)** are used which preferably have different temperatures for processing the intermediate products. For the first reactor **15** it is preferred that it has a volume of 10 mL to 60 mL and more preferably of 16 to 32 mL, Omnifit, outside diameter (OD) 1/16 in and inside diameter (ID) 0.8 mm). This first reactor **15** is preferably kept at room temperature.

Moreover, the inventive continuous flow reactor may further comprise a second reactor **16** downstream to the first reactor **15.** This second reactor **16** is preferably kept at a temperature between 50°C and 70°C and more preferably at about 60°C. For the optional second reactor **16** it is preferred that it is a 10 mL reactor from Vapourtec, R4 unit. Any device able to heat a reactor to a temperature between 50°C and 70°C and preferably to 60 °C could be used instead of the Vapourtec R4 unit, for example, other commercially available continuous flow systems, oil bath or water bath with an heating plate.

In another embodiment of the present invention three reactors **(15, 16, 17)** are used which preferably have different temperatures for processing the intermediate products. For the first reactor **15** it is preferred that it has a volume of 5 mL to 20 mL and more preferably of 5 mL to 8 mL, Omnifit, outside diameter (OD) 1/16 in and inside diameter (ID) 0.8 mm). This first reactor **15** is preferably cooled to 0°C. The inventive continuous flow reactor may further comprise a second reactor **16** downstream to the first reactor. For the optional second reactor **16** it is preferred that it is a 10 mL to 60 mL reactor and more preferably a 25 mL to 30 mL reactor. This second reactor **16** is preferably used at room temperature. Moreover, the inventive continuous flow reactor may further comprise a third reactor **17** downstream to the second reactor **16.** This third reactor 17 is preferably kept at a temperature between 50°C and 70°C and more preferably at about 60°C. For the optional third reactor **17** it is preferred that it is a 5 mL to 60 mL reactor and more preferably a 10 mL reactor such as from Vapourtec, R4 unit. Any device able to heat a reactor at about 60°C could be used instead of the Vapourtec R4 unit, for example: other commercially available continuous flow systems, oil bath or water bath with an heating plate.

The optional back-pressure regulator needs to be acid resistant and is preferably from Vapourtec. It operates preferably at 2.2 bar, but is adjustable for the inventive method in a range from 0 to 8 bar. Any acid resistant back-pressure operating in that range of pressure could be used instead of the one provided by Vapourtec.

Thus, the present invention is also related to artemisinin (6) produced or synthesized according to the synthesis disclosed herein. More preferably the present invention is directed to artemisinin (6) produced or synthesized according to the synthesis disclosed herein using the photochemical reactor or the continuous flow reactor as disclosed herein.

Optionally, the inventive continuous flow reactor may further comprise an automated two inlet switch valve **13a**

for regulating the composition of the feed **F1** for the solution of dihydroartemisinic acid (2), allowing for rapid switching from pure solvent to the solution containing the dissolved dihydroartemisinic acid (2). A similar switch valve **13b** can be disposed in the feed **F2** of the acidic solution (for instance a TFA solution) in order to regulate the composition by portioning the stem solution of the acidic solution and the respective solvent. Any valves could be used, as long as they are resistant against corrosion and acids.

In Fig. 1 a schematic drawing of an inventive photochemical reactor is depicted. The photochemical reactor is the central piece of the continuous flow reactor according to the invention. In the center of the photochemical reactor is a light source, preferably a Hg lamp. The light source has an oblong shape. Its longitudinal axis is vertical. However, the photochemical reactor could also be used in the horizontal orientation, i.e. where its longitudinal axis is horizontal. The lamp is disposed centrally in an immersion well that surrounds the lamp in a cylinder-like shape. At a certain distance (for instance, the outer diameter of the immersion well is 4.83 cm and the inner diameter of the pyrex filter is 4.85 cm, so the distance between the two should be 0.01 cm) a further cylinder-like shape surrounds the immersion well, having the same central point. This structure is a filter, preferably a Pyrex filter. Around this filter multiple loops of tubing are wrapped in a spiral like form. Thus the outer diameter of the filter determines the inner diameter if these loops. The number of loops can be variable. The distance between the loops is preferably equidistant but this is not compulsory. There are preferably two layers of loops, the first layer (the closest to the lamp) is made of, for instance, approximately 135 loops and the second layer of, for instance, approximately of 115 loops, so that the complete tubing consists of approximately 250 loops. However, the tubing preferably consists of 100 to 1000 loops in one, two or three layers, preferably of 150 to 500 loops in one, two or three layers, more preferably of 200 to 400 loops in one, two or three layers, still more preferably of 220 to 300 loops in one, two or three layers, and most preferably of 230 to 270 loops in one, two or three layers. Moreover two or three layers of loops and more preferably two layers of loops are preferred. It is also preferred that there is no distance between the loops. At the upper end of the tubing there is a connection to a mixer in which dihydroartemisinic acid and oxygen are mixed. At the lower end of the tubing there is an outlet for the reaction products for further processing. For this process the addition of an acidic solution such as TFA solution is needed. This addition can take place in the mixer together with the dihydroartemisinic acid and the oxygen, or alternatively when the products have left the photoreactor.

Fig. 2 shows the continuous flow reactor setup for the synthesis of artemisinin (6) starting from dihydroartemisinic acid (2). The continuous flow reactor for the synthesis of artemisinin (6) consists of two pumps **1a** and **1b,** a mass flow controller **2** connected to a manometer 3 fixed on an oxygen tank **4,** a check-valve **5a** between the pump **1a** and the mixer **6a,** and another check valve 5b between the mass flow controller **2** and the mixer **6a.** The center piece of the continuous flow reactor is a photoreactor with multiple loops of a tubing **7** wrapped tightly around a filter **8,** which surrounds an immersion well **9** cooled by a thermostat **10,** a light source **11,** a power supply **12** for the photochemical lamp **11,** a second mixer **6b,** a reactor **15,** preferably a second reactor **16,** preferably a third reactor **17** and a collection flask **18.** The flow reactor setup comprises preferably in addition an automated two inlet valve **13** allowing for rapid switching from pure solvent to the solution containing the dissolved reagents at the intake pump and optionally a back-pressure regulator **14** which was installed in order to increase the internal pressure of the system. All the parts are connected with tubing.

FEP tubing was preferably selected for its high transmittance and stability in the UV-vis light range, its flexibility and its high chemical resistance. The 2 mm thick Pyrex filter was advantageous to absorb wavelengths below 300 nm, to prevent degradation of the tubing, and to avoid any undesired side reactions involving short wavelength light. The temperature in the tube during the reaction is estimated to range preferably from 25 to 30°C, based on temperature of the cooling liquid, which circulate in the immersion well, between the lamp and the filter. For safety reasons, the lamp was placed inside an aluminum box for blocking UV irradiation. Two fans can optionally be installed for additional cooling.

A solution of dihydroartemisinic acid (2) and the photosensitizer in dichloromethane was mixed (2.5 mL/min) with a flow of oxygen gas (7.5 mL/min) and passed through the above described photoreactor. The residence time in the reactor is approximately 2.0 minutes. Using a Vapourtec R2C+ pump, a solution of TFA in toluene was added at a flow rate of 0.5 mL/min with the outlet stream of the photoreactor containing mainly intermediate (3) to induce the acid catalyzed Hock cleavage. A PTFE reactor (having two compartments: a 16 mL volume kept at room temperature and a 10 mL volume heated at about 60°C) resulted in approximately 2.5 min residence time to achieve also the reaction of triplet oxygen and further condensation. After a total residence time of 4.5 min the product stream containing mainly desired artemisinin (6) was obtained. Purification by chromatography was performed. The overall yield of (6) in the multistep reaction from dihydroartesiminic acid (2) is 39% with a productivity of 200 g of the anti-malaria compound per day. A rough calculation based on 300 million doses of artemisinin needed per year reveals that approximately 2050 photoreactors are needed to meet the demand, even if no further improvements are made to the process described here.

The continuous flow process of the present invention provides a highly complex natural product from a much less complex molecule that can be isolated in larger quantities or can be readily produced in yeast. The efficiency, simplicity and productivity of the approach will provide access to much needed medication against malaria. Further process optimization may result in even higher yields and productivities but even at this time the process disclosed here should meet the ever growing demand for low cost artemisinin to treat malaria sufferers around the world.

**Description of Figures**

Fig. 1: Schematic drawing of the photoreactor for the synthesis of artemisinin

Fig. 2: System diagram of the continuous flow reactor for the synthesis of artemisinin

Fig. 3: $^1$H NMR of the produced artemisinin

Fig. 4: Flow reactor setup for the synthesis of artemisinin

**Examples**

Methods:

$^1$H NMR spectra were recorded on a Varian 400-MR spectrometer (at 400 MHz) at ambient temperature. The proton signal of residual non-deuterated solvent (δ 7.26 ppm for $CHCl_3$) was used as an internal reference for $^1$H spectra. Data are reported as follows: chemical shift in parts per million (δ, ppm), multiplicity (s = singlet, d = doublet, t = triplet, q = quartet, qn = quintet, m = multiplet and br = broad), coupling constant reported in Hertz (Hz) and integration. $^{13}$C spectra were recorded on a Varian 400-MR spectrometer (at 101 MHz) at ambient temperature. Chemical shifts are reported in parts per million (δ, ppm). The carbon signal of deuterated solvent (δ 77.16 ppm for $CDCl_3$) was used as an internal reference for $^{13}$C spectra.

Infrared (IR) spectra were recorded as thin films on a Perkin-Elmer 1600 FTIR spectrophotometer. Melting points were recorded using an Electrothermal IA 9300 melting point apparatus and are uncorrected. Optical rotations (OR) were measured with a Schmidt & Haensch Unipol L 1000 at a concentration (c) expressed in g/100 mL. High-resolution mass spectra (HRMS) were recorded with an Agilent 6210 ESI-TOF mass spectrometer at the Freie Universität Berlin, Mass Spectrometry Core Facility.

Analytical thin layer chromatography (TLC) was performed on Kieselgel 60 F254 glass plates pre-coated with a 0.25 mm thickness of silica gel. The TLC plates were visualized with UV light and by staining with an aqueous solution of potassium permanganate ($KMnO_4$) or a mixture of iodine and silica. Column chromatography was performed using Kieselgel 60 (230-400 mesh) silica gel with a typical 50-100: 1 weight ratio of silica gel to crude product.

Example 1: Reaction conditions for oxidation of dihydroartemisinic acid (2)

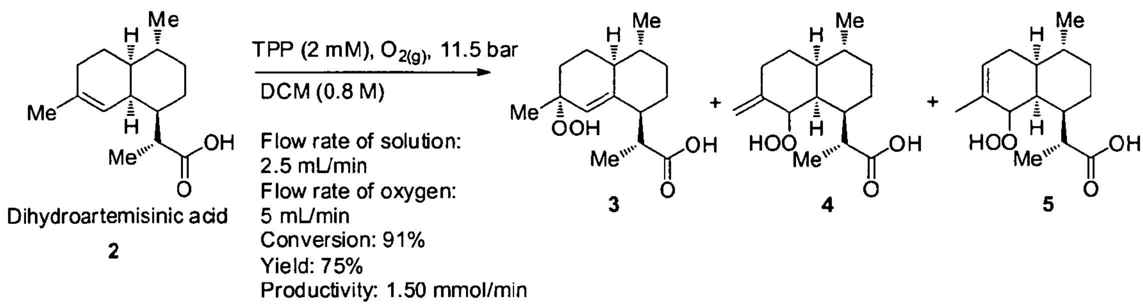


Dihydroartemisinic acid **2** + **3** + **4** + **5**

A solution of dihydroartemisinic acid (0.95 g, 4.0 mmol) and tetraphenylporphyrin (6.1 mg, 10 μmol) in dichloromethane (total volume of the solution: 5.0 mL, volumetric flask) was prepared. The lamp was turned on 30 min prior to the beginning of the experiment. The reactor was flushed with pure dichloromethane (2.5 mL/min) and oxygen (5 mL/min, 11.5 bar) for 5 min. The reagents were then injected at a flow rate of 2.5 mL/min and the flow of oxygen was readjusted to 5 mL/min (11.5 bar). After the injection of the entire solution of dihydroartemisinic acid, the reactor was flushed with pure dichloromethane (2.5 mL/min) to recover all the material. The crude material was concentrated under reduced pressure to remove the dichloromethane affording a mixture of the intermediate products (3), (4) and (5) and TPP as a green solid (1.0856 g). Part of this mixture (200 mg) was dissolved in $CDCl_3$ and mesitylene (103 μL, 0.741

mmol, 98% pure) was added. $^1H$ NMR analysis showed a conversion of 91 % and a yield of the desired product of 75%.

Example 2: Reaction conditions for oxidation of dihydroartemisinic acid (2) in flow and cleavage of tertiary allylic peroxide (3) in batch to obtain artemisinin (6)

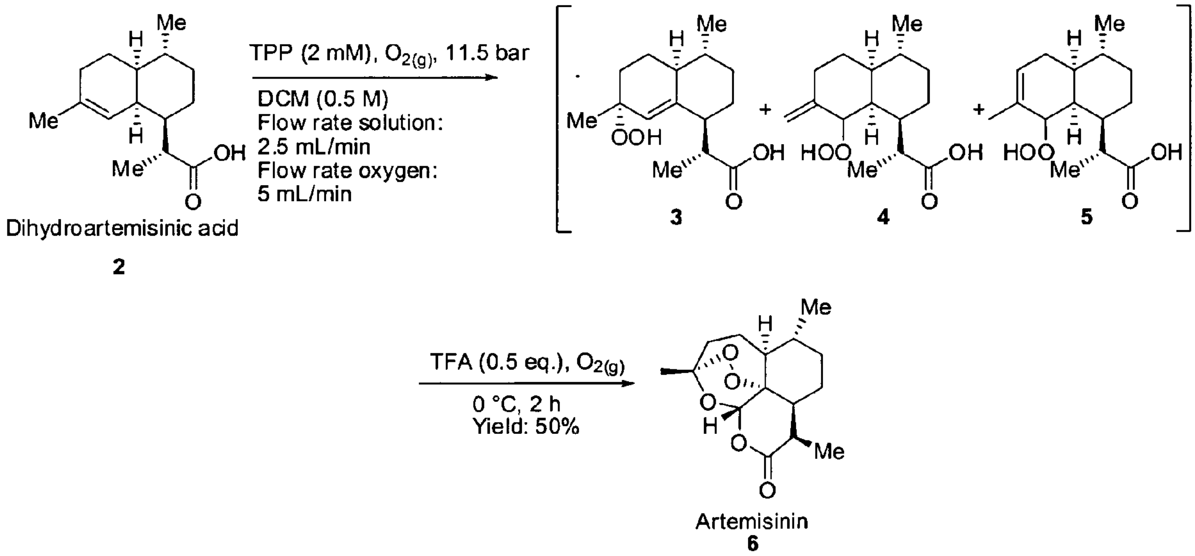


Dihydroartemisinic acid
2

3 4 5

Artemisinin
6

A solution of dihydroartemisinic acid (4) (1.18 g, 5.0 mmol) and tetraphenylporphyrin (12 mg, 20 μmol) in dichloromethane (total volume of the solution: 10.0 mL, volumetric flask) was prepared. The lamp was turned on 30 min prior to the beginning of the experiment. The reactor was flushed with pure dichloromethane (2.5 mL/min) and oxygen (5 mL/min, 11.5 bar) for 5 min. The reagents were then injected at a flow rate of 2.5 mL/min and the flow of oxygen was readjusted to 5 mL/min (11.5 bar). After the injection of the entire solution of (4), the reactor was flushed with pure dichloromethane (2.5 mL/min) to recover all material. The reaction mixture was collected in a round bottom flask and was cooled down to 0 °C. Oxygen was bubbled into the reaction mixture at atmospheric pressure. After 2 min of bubbling, TFA (0.19 mL, 2.5 mmol, 0.5 eq.) was added drop wise. The resulting mixture was stirred at 0 °C for 2 h, while maintaining the oxygen bubbling. Then, the reaction was quenched with a saturated aqueous solution of $NaHCO_3$. The resulting biphasic mixture was stirred at room temperature until disappearance of the green colour. The phases were separated and the aqueous phase was extracted with dichloromethane (3 times). The combined organic layers were dried over anhydrous $Na_2SO_4$, filtered and concentrated under reduced pressure. Purification over silica gel (5% - 20% EtOAc in cyclohexane) afforded artemisinin (0.707 g, 50%) as a yellow solid.

Example 3: Reaction conditions for oxidation of dihydroartemisinic acid (2) in continuous flow, followed by the cleavage of tertiary allylic peroxide (3) in continuous flow to obtain artemisinin (6) (sequential process)

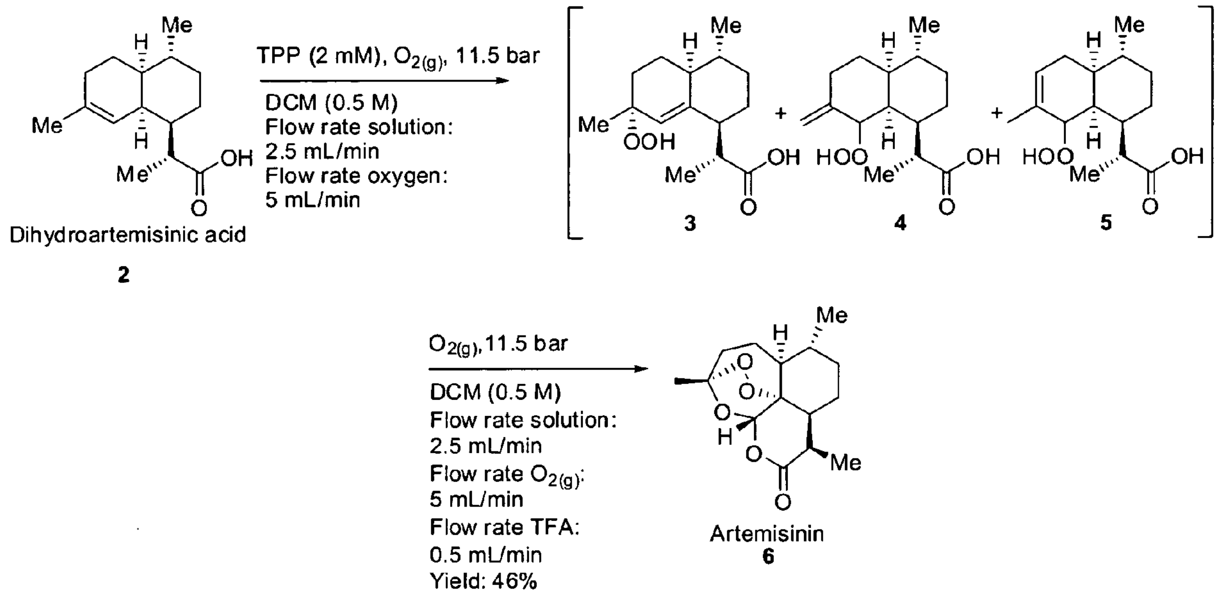


Dihydroartemisinic acid 2

3 + 4 + 5

Artemisinin 6

A solution of dihydroartemisinic acid (1.18 g, 5.0 mmol) and tetraphenylporphyrin (12 mg, 20 μmol) in dichloromethane (total volume of the solution: 10 mL, volumetric flask) was prepared. The lamp was turned on 30 min prior to the beginning of the experiment. The reactor was flushed with pure dichloromethane (2.5 mL/min) and oxygen (5 mL/min, 11.5 bar) for 5 min. The reagents were then injected at a flow rate of 2.5 mL/min and the flow of oxygen was readjusted to 5 mL/min (11.5 bar). After the injection of the entire solution of (1), the reactor was flushed with pure dichloromethane (2.5 mL/min) to recover all the material. The crude material was concentrated under reduced pressure to remove the dichloromethane affording a mixture of (3), (4), (5) and TPP as a green solid.

The crude mixture was dissolved in DCM (total volume of the solution: 10 mL, volumetric flask). The crude mixture was injected at a flow of 2.5 mL/min and combined with oxygen (5 mL/min, 11.5 bar) and a solution of TFA (1.9 mL of TFA in 18.1 mL of DCM) at a flow rate of 0.5 mL/min with the help of a cross mixer. The reaction mixture was passed a PTFE reactor composed of 32 mL loop at rt and 10 mL loop heated at 60°C. The crude material was collected in a flask containing a saturated aqueous solution of $NaHCO_3$. The resulting biphasic mixture was stirred at room temperature until green colour disappeared. The phases were separated and the aqueous phase was extracted three times with dichloromethane. The combined organic layers were dried over anhydrous $Na_2SO_4$, filtered and concentrated under reduced pressure. Purification over silica gel (5% - 20% EtOAc, in cyclohexane) afforded artemisinin (0.66 g, 46%) as a yellow solid.

Example 4: Reaction conditions for the synthesis of artemisinin (6) in continuous flow

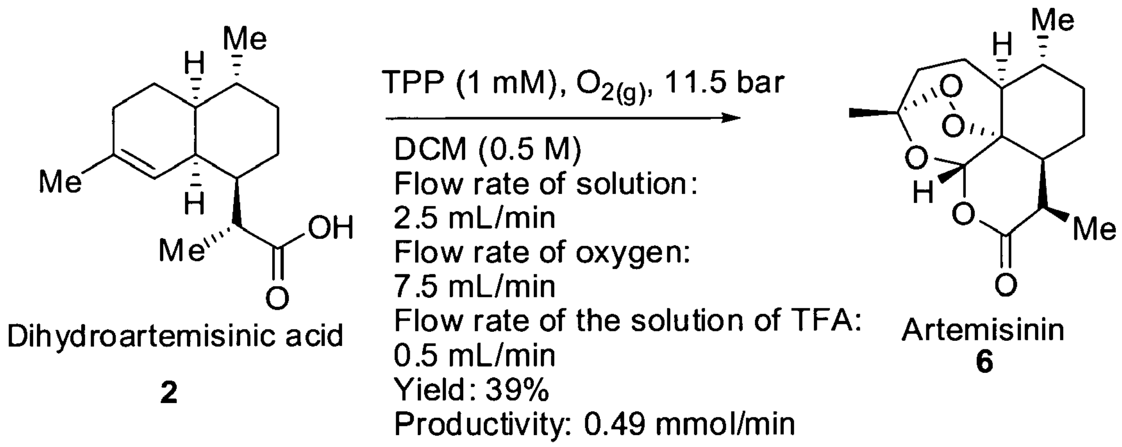


Dihydroartemisinic acid **2**

Artemisinin **6**

A solution of dihydroartemisinic acid (2.95 g, 12.5 mmol) and tetraphenylporphyrin (15 mg, 0.02 mmol) in dichloromethane (total volume of the solution: 25 mL, volumetric flask) and a solution of trifluoroacetic acid (1.9 mL, 25 mmol) in dichloromethane (18.1 mL) were prepared and given into their respective feed. The Hg lamp was turned on 30 min prior to the beginning of the experiment and the second portion of the photoreactor was heated at 60°C. The photoreactor was flushed with pure dichloromethane (2.5 mL/min), dichloromethane (0.5 mL/min) and oxygen (7.5 mL/min, 11.5 bar) for 10 min. The reagents were then injected via their respective feed at a flow rate of 2.5 mL/min and the oxygen flow was readjusted to 7.5 mL/min (11.5 bar). Both streams joined in the first mixer. From there they entered the photoreactor. The TFA solution was injected at the exit of the photoreactor into a second mixer at a flow rate of 0.5 mL/min and the resulting mixture was pushed into the thermal reactor. The crude material containing the produced artemisinin was collected in a flask containing a saturated aqueous solution of $NaHCO_3$. The resulting biphasic mixture was stirred at room temperature until the green color disappeared. Phases were separated and the aqueous phase was extracted with dichloromethane (3 times). The combined organic layers were dried over anhydrous $Na_2S0_4$, filtered and concentrated under reduced pressure. Purification over silica gel (5% - 20% EtOAc, in cyclohexane) afforded artemisinin (1.36 g, 39%) as a off-white solid. Further purification by recrystallization in cyclohexane afforded white needles. $^1H$ NMR (400 MHz, $CDCl_3$) $\delta$ 5.86 (s, 1H), 3.40 (dq, J = 7.3, 5.4 Hz, 1H), 2.47-2.39 (m, 1H), 2.08-1.98 (m, 2H), 1.91-1.86 (m, 1H), 1.81-1.74 (m, 2H), 1.51-1.34 (m, 3H), 1.45 (s, 3H), 1.21 (d, J = 7.3 Hz, 3H), 1.11-1.04 (m, 2H), 1.00 (d, J = 6.0 Hz, 3H). The $^1H$ NMR spectrum of the obtained artemisinin (6) is shown in Fig. 3. Mp = 153-154 °C. $[\alpha]_D^{20}$ +66.3 ° (c 0.97, $CHCl_3$). $^{13}C$ NMR (101 MHz, $CDCl_3$) $\delta$ 172.2, 105.5, 93.9, 79.6, 50.2, 45.1, 37.7, 36.1, 33.8, 33.0, 25.4, 25.0, 23.6, 19.9, 12.7. IR (film) $\nu$ 2960, 2933, 2860, 1731, 1112, 991 $cm^{-1}$. HRMS calcd for $C_{15}H_{22}O_5$ (M+) 282.1467, found 282.1463. MS (EI) m/z 282 (1) [$M^+$], 250 (5), 192 (70), 150 (40), 55 (63), 43 (100). Spectral data were in agreement with published data (Yadav, J. S.; Satheesh, B.; Sabitha, G. Tetrahedron Lett. 2003, 44, 387-389.).

Example 5: Flow reactor setup for the synthesis of artemisinin according to example **4**

The flow reactor setup for the synthesis of artemisinin (6) consists of a feed **F1** for a solution of dihydroartemisinic acid (2), an automated two inlet switch valve **13a** for regulating the composition of the feed for the solution of dihydroartemisinic acid (2), allowing for rapid switching from pure solvent to the solution containing the dissolved dihydroartemisinic acid, a first HPLC pump **1a** (Vapourtec, R2C+ unit) downstream to switch valve **13a,** pumping the dihydroartemisinic acid (2) solution with a throughput of 2.5 mL/min to the first ETFE T-mixer **6a** (IDEX Health and Science, P-632) for mixing the dihydroartemisinic acid (2) solution and the oxygen, a first check-valve **5a** (IDEX Health and Science, inline check-valve CV-3010) between the first HPLC pump **1a** and the mixer **6a,** a mass flow controller **2** (Influx, SV1B5-AI05, allowing control of the oxygen flow rate from 5-90 $cm^3$/min) connected to a manometer **3** fixed on an oxygen tank **4** (Air Liquide, $O_2$ 99.995% pure), thus generating a steady oxygen flow of 7.5 mL/min, another check valve **5b** (IDEX Health and Science, inline check-valve CV-3010) between the mass flow controller **2** and the first mixer **6a,** multiple loops of FEP tubing 7 (20 mL, IDEX Health & Science, fluorinated ethylene polymer 1520, natural color, outside diameter (OD) 1/16 in and inside diameter (ID) 0.030 in) wrapped tightly around a Pyrex filter **8** (inner diameter 4.5 cm and wall thickness 0.2 cm) which surrounds the quartz immersion well **9** cooled by a thermostat **10** (Huber, Unistat 360), a medium pressure

Hg lamp **11** (Ace Glass, UV 450 immersion lamp, 5 in arc, radial lead, 7825-34), a power supply **12** for photochemical lamp **11** (Ace Glass, 7830), a second ETFE T-mixer **6b** IDEX Health and Science, P-632), a first PTFE reactor **15** (11 mL, Omnifit, outside diameter (OD) 1/16 in and inside diameter (ID) 0.8 mm), a second PTFE reactor at room temperature **16** (5 mL, Vapourtec), a third heated (60°C) PTFE reactor **17** (10 mL, Vapourtec, R4 unit) and a collection flask **18** for collecting the synthesized artemisinic acid. A feed **F2** for the TFA solution is regulated via an automated two inlet switch valve **13b** for regulating the composition of the feed for the TFA solution, allowing for rapid switching from pure solvent to the TFA solution. A second HPLC pump **1b** (Vapourtec, R2C+ unit) pumps TFA with a throughput of 0.5 mL/min to into the second mixer 6b disposed at the outlet of the tubing **7** of the photoreactor. There the TFA is reacted with the products of the photoreactor process. A back-pressure regulator **14** of 2.2 bar (Vapourtec) was installed in order to increase the internal pressure of the system. FEP tubing was selected for its high transmittance and stability in the UV-vis light range, its flexibility and its high chemical resistance. The 2 mm thick Pyrex filter was essential to absorb wavelengths below 300 nm, to prevent degradation of the tubing, and to avoid any undesired side reactions involving short wavelength light. The temperature in the tube during the reaction is estimated to range from 25 to 30°C, based on temperature measurements taken between the cooling jacket and the tube. For safety reasons, the lamp was placed inside an aluminum box for blocking UV irradiation. Two fans were installed for additional cooling.

## Claims

**1.** A method for producing artemisinin from dihydroartemisinic acid comprising the following steps:

A) providing dihydroartemisinic acid represented by the following formula

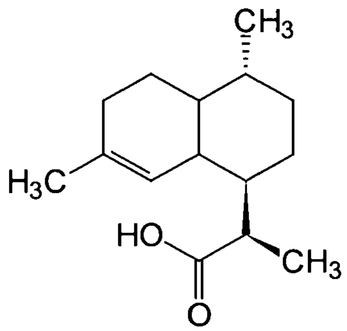

B) performing in a continuous flow reactor the following reactions

i) photooxidation of dihydroartemisinic acid with singlet oxygen,
ii) followed by an acid mediated cleavage and
iii) subsequent oxidation with triplet oxygen
in order to obtain artemisinin of the following formula:

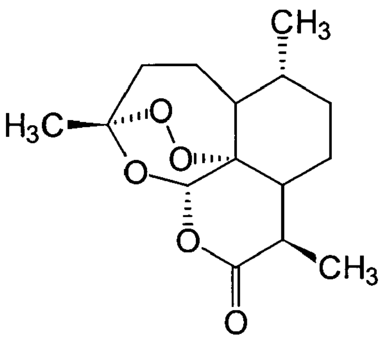

**2.** The method according to claim 1, wherein the dihydroartemisinic acid is obtained by reducing artemisinic acid of

the following formula

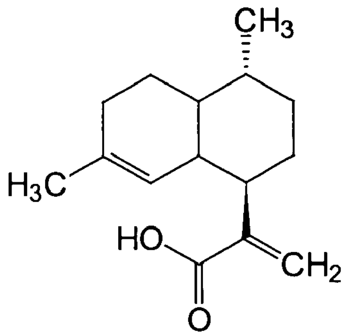

to dihydroartemisinic acid.

3. The method according to claim 1 or 2, wherein the continuous flow reactor comprises a tube made of a fluorinated or perfluorinated alkylene polymer wherein the photooxidation of dihydroartemisinic acid takes place and which is wrapped around a photochemical reactor containing a light source.

4. The method according to claim 1, 2 or 3, wherein for the acid mediated cleavage trifluoroacetic acid is used.

5. The method according to any previous claim, wherein 12.5 mmol dihydroartemisinic acid are processed in a total residence time below 5 minutes through the continuous flow reactor in order to yield 39% artemisinin.

6. A photochemical reactor for the production of artemisinin from dihydroartemisinic acid comprising

- a light source **11,**
- an immersion well 9 surrounding the light source **11,**
- a filter **8** surrounding the light source **11** and
- multiple loops of a tubing **7** wrapped tightly around the filter **8,** the tubing **7** having an inlet for a mixture of dihydroartemisinic acid and oxygen on its one end and an outlet for the reacted products on the opposite end.

7. The photochemical reactor according to claim 6, further comprising a thermostat **10** for cooling the immersion well **9.**

8. A continuous flow reactor for the production of artemisinin from dihydroartemisinic acid comprising

- the photochemical reactor according to claim 6 or 7,
- a first mixer **6a,** connected to the inlet of the tubing 7 of the photochemical reactor,
- a feed **F1** for a solution of dihydroartemisinic acid,
- a first pump **1 a** for pumping the solution of dihydroartemisinic acid to the first mixer **6a,**
- a check-valve **5a** between the first pump **1a** and the first mixer **6a,**
- an oxygen tank **4** with a manometer **3,**
- a mass flow controller **2** disposed between the oxygen tank **4** and the first mixer **6a** for controlling the oxygen flow rate,
- a check valve **5b** between the mass flow controller **2** and the first mixer **6a,**
- a feed **F2** for an acidic solution,
- a second mixer **6b** connected to the outlet of the tubing **7** of the photochemical reactor and to the feed **F2** of the acidic solution,
- a second pump **1 b** for pumping the acidic solution to the second mixer **6b,**
- at least one reactor **15** for producing or completing the synthesis of artemisinin, connected downstream to the second mixer **6b,** and
- a collection flask **18** for collecting the artemisinin-containing solution from at least one reactor **15.**

9. The continuous flow reactor according to claim 8, wherein the acidic solution flows from its feed **F2** not to the second mixer **6b** but to the first mixer **6a** and the second pump **1 b** is not arranged between the feed **F2** for the acidic solution

and the second mixer **6b** but between the feed **F2** for the acidic solution and the first mixer **6a.**

**10.** The continuous flow reactor according to claim 8 or 9, further comprising

- a second reactor **16** downstream to the first reactor **15** or
- a second reactor **16** downstream to the first reactor **15** and
- a third reactor **17** downstream to the second reactor **16.**

**11.** The continuous flow reactor according to any of claims 8 to 10, further comprising

- an automated two inlet switch valve **13a** for regulating the composition of the feed **F1** for the solution of dihydroartemisinic acid allowing for rapid switching from pure solvent to the solution containing the dissolved dihydroartemisinic acid and/or
- an automated two inlet switch valve **13b** for regulating the composition of the feed **F2** for the acidic solution allowing for rapid switching from pure solvent to the acidic solution.

**12.** The continuous flow reactor according to any of claims 8 to 11, further comprising

- a back-pressure regulator **14** downstream to the last of the reactors **15, 16** or **17.**

**13.** Artemisinin produced according to the synthesis of claim 1 or 2.

Figure 1

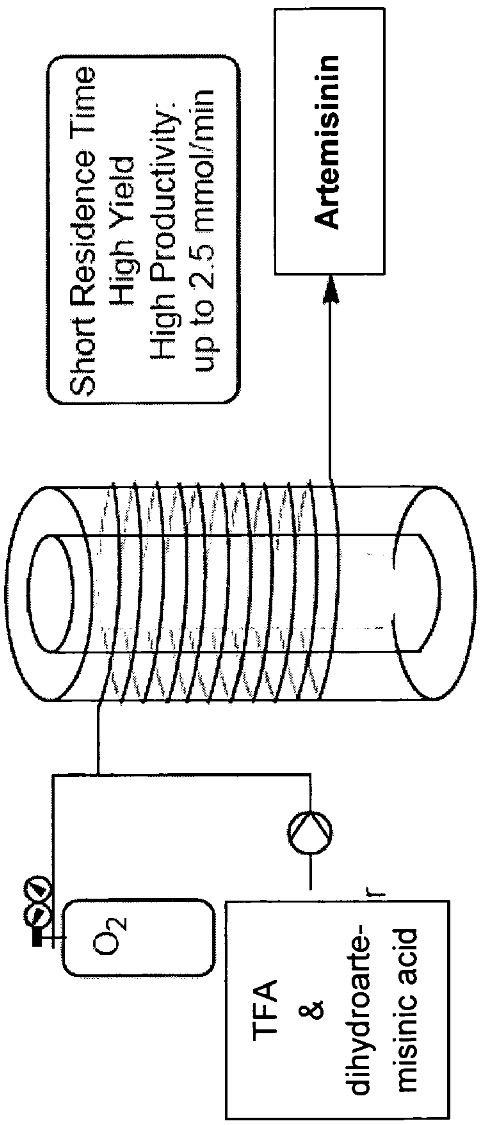
TFA
&
dihydroarte-
misinic acid
O2
Short Residence Time
High Yield
High Productivity:
up to 2.5 mmol/min
Artemisinin

Figure 2

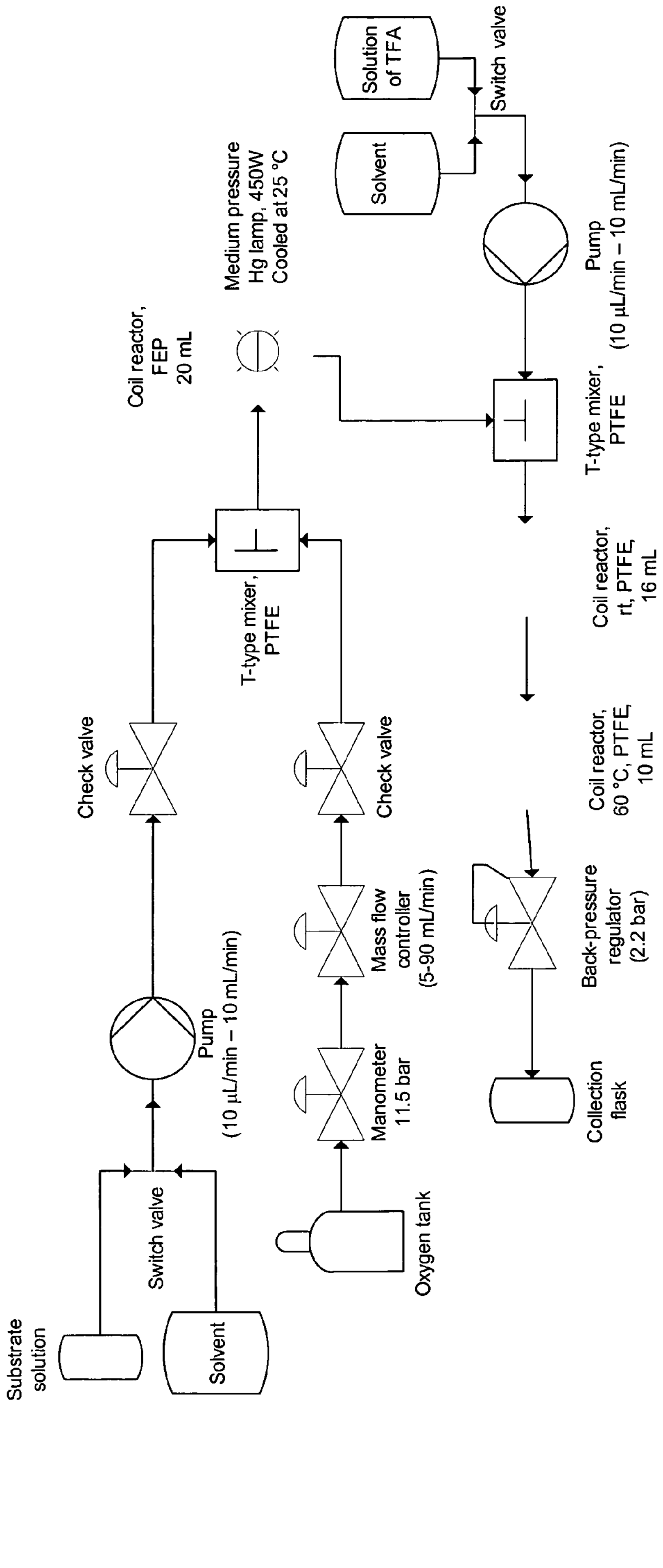
Substrate solution
Solvent
Switch valve
Pump (10 µL/min – 10 mL/min)
Check valve
Oxygen tank
Manometer 11.5 bar
Mass flow controller (5-90 mL/min)
Check valve
T-type mixer, PTFE
Coil reactor, FEP 20 mL
Medium pressure Hg lamp, 450W Cooled at 25 °C
Solvent
Solution of TFA
Switch valve
Pump (10 µL/min – 10 mL/min)
T-type mixer, PTFE
Coil reactor, rt, PTFE, 16 mL
Coil reactor, 60 °C, PTFE, 10 mL
Back-pressure regulator (2.2 bar)
Collection flask

Figure 3

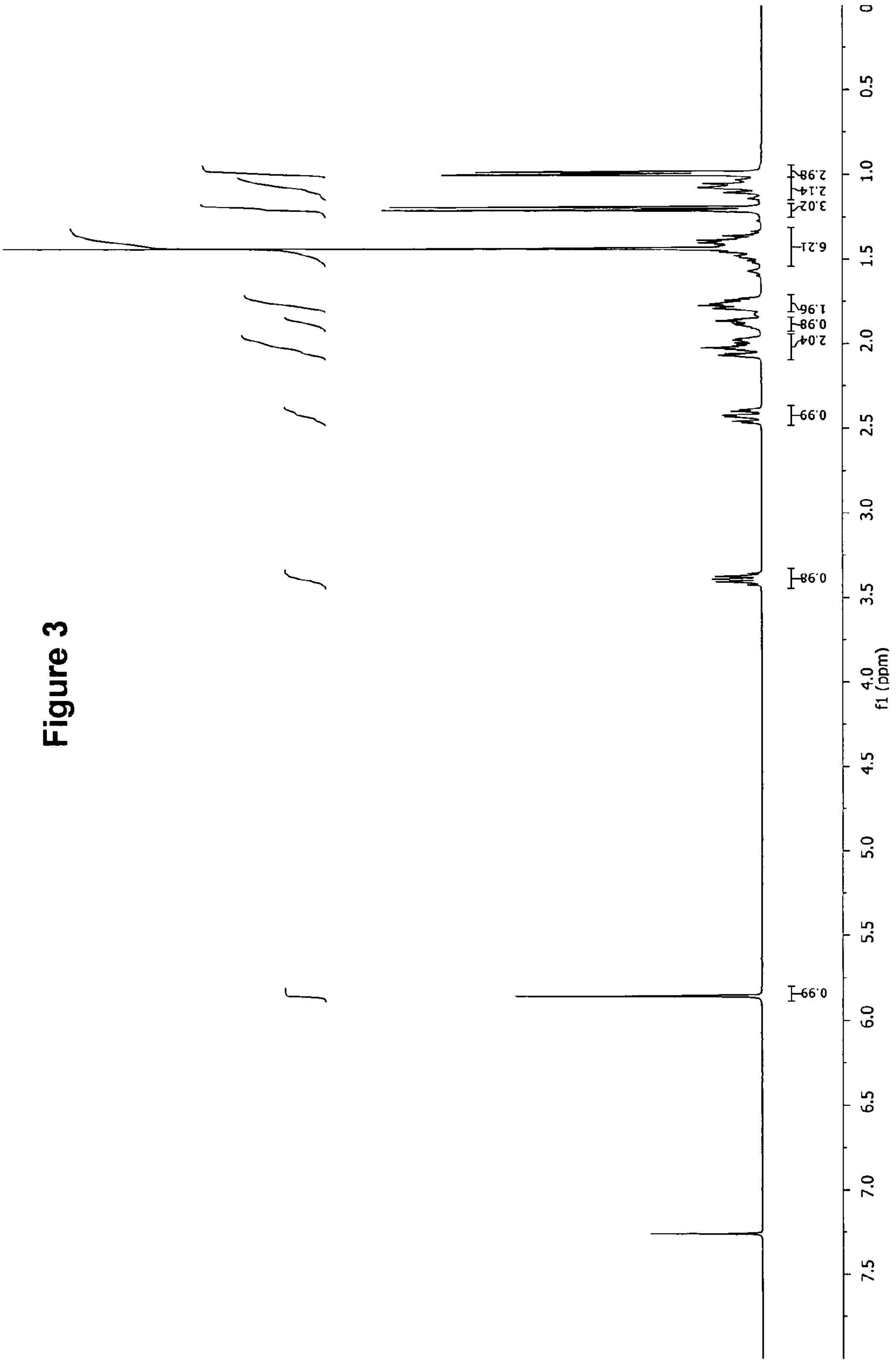
2.98
2.14
3.02
6.21
1.96
0.98
2.04
0.99
0.98
0.99
0
0.5
1.0
1.5
2.0
2.5
3.0
3.5
4.0
4.5
5.0
5.5
6.0
6.5
7.0
7.5
f1 (ppm)

## Figure 4

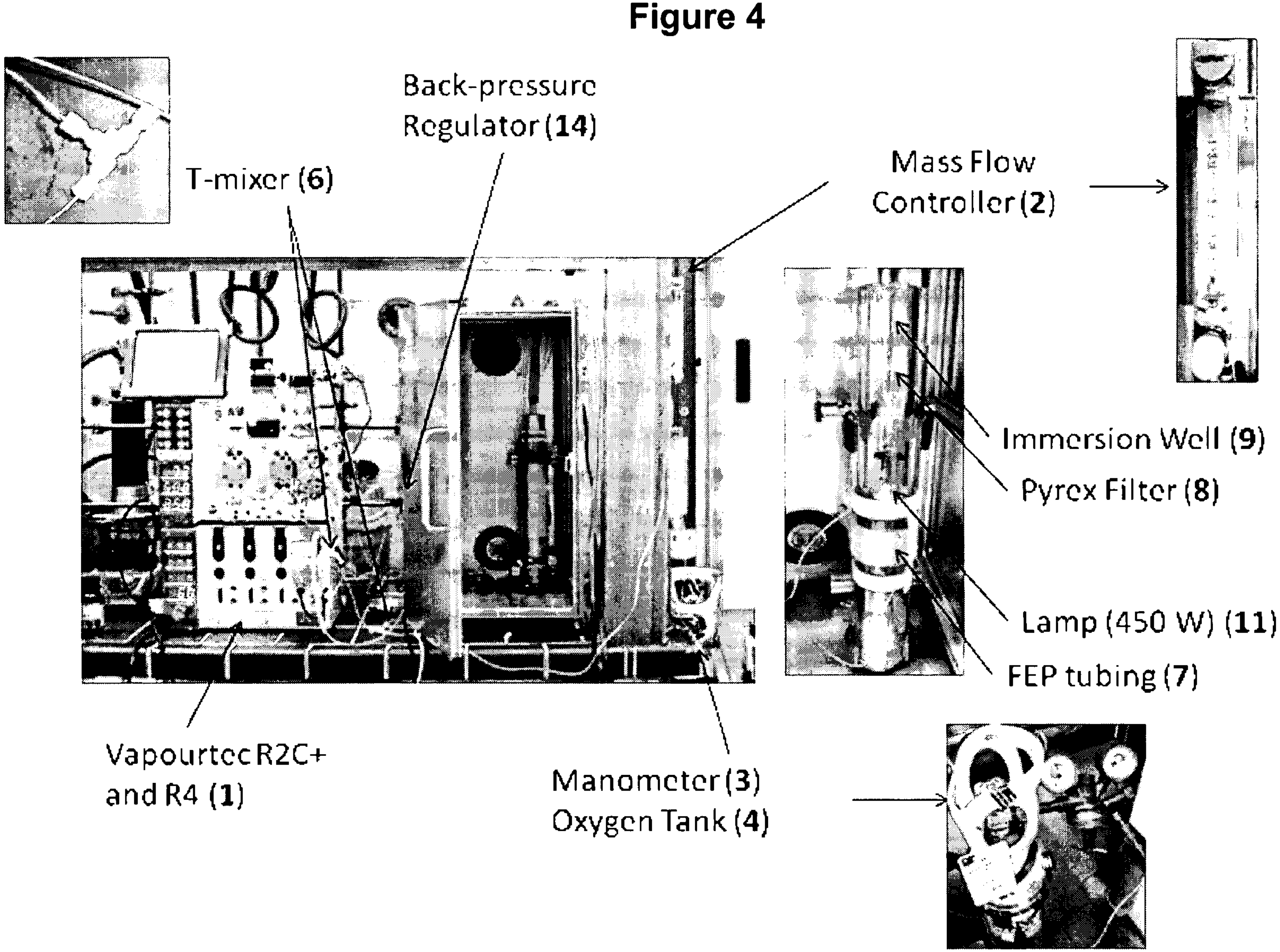
T-mixer (6)
Back-pressure Regulator (14)
Mass Flow Controller (2)
Immersion Well (9)
Pyrex Filter (8)
Lamp (450 W) (11)
FEP tubing (7)
Vapourtec R2C+ and R4 (1)
Manometer (3)
Oxygen Tank (4)

Europäisches Patentamt
European Patent Office
Office européen des brevets

# EUROPEAN SEARCH REPORT

Application Number
EP 11 00 7018

| Category | Citation of document with indication, where appropriate, of relevant passages | Relevant to claim | CLASSIFICATION OF THE APPLICATION (IPC) |
|---|---|---|---|
| X | NANCY ACTON AND RONALD J ROTH: "On the Conversion of Dihydroartemisinic Acid into Artemisinin", JOURNAL OF ORGANIC CHEMISTRY, AMERICAN CHEMICAL SOCIETY, EASTON.; US, vol. 57, 1 January 1992 (1992-01-01), pages 3610-3614, XP007919840, ISSN: 0022-3263 | 13 | INV. C07D493/22 |
| Y | * page 3614 * | 1,3-12 | |
| Y,D | WO 2011/030223 A2 (SANOFI AVENTIS [FR]; KRAFT VOLKER [DE]; KRETZSCHMAR GERHARD [DE]; ROSS) 17 March 2011 (2011-03-17) * claim 1 * | 2 | |
| Y | BENJAMIN D A HOOK ET AL: "A Practical Flow Reactor for Continuous Organic Photochemistry", JOURNAL OF ORGANOMETALLIC CHEMISTRY, ELSEVIER-SEQUOIA S.A. LAUSANNE, CH, vol. 70, 1 January 2005 (2005-01-01), pages 7558-7564, XP007919839, ISSN: 0022-328X, DOI: 10.1021/JO050705P [retrieved on 2006-08-13] * page 7559; figure 2 * | 1-12 | TECHNICAL FIELDS SEARCHED (IPC): C07D |

1 The present search report has been drawn up for all claims

| Place of search | Date of completion of the search | Examiner |
|---|---|---|
| Munich | 1 December 2011 | Lewis, Sara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 00 7018

This annex lists the patent family members relating to the patent documents cited in the above-mentioned European search report.
The members are as contained in the European Patent Office EDP file on
The European Patent Office is in no way liable for these particulars which are merely given for the purpose of information.

01-12-2011

| Patent document cited in search report | Publication date | Patent family member(s) | Publication date |
|---|---|---|---|
| WO 2011030223 A2 | 17-03-2011 | US 2011230669 A1 | 22-09-2011 |
| | | WO 2011030223 A2 | 17-03-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009088404 A1
- US 4992561 A
- WO 2006128126 A1, Reiling, K. K.; Renninger, N. S.; McPhee, D. J.; Fisher, K. J.; Ockey, D. A.
- WO 2011030223 A2


### Non-patent literature cited in the description


- *World Malaria Report 2010,* 2010
- **WHITE, N. J.** *Science,* 2008, vol. 320, 330-334
- **BROWN, G. D.** *Molecules,* 2010, vol. 15, 7603-7698
- **RO, D.-K. et al.** *Nature,* 2006, vol. 440, 940-943
- **SCHWEITZER, C. ; SCHMIDT, R.** *Chem. Rev.,* 2003, vol. 103, 1685-1758
- **HOFFMANN, N.** *Chem. Rev.,* 2008, vol. 108, 1052-1103
- **ZHANG, Y. et al.** *J. Biol. Chem.,* 2008, vol. 31, 21501-21508
- **RO, D.K. et al.** *Nature,* 2006, vol. 440, 940-943
- **ROTH, R. J. ; ACTON, N. J.** *Chem. Edu.,* 1991, vol. 68, 612-613
- **ROTH, R. J. ; ACTON, N. A.** *J. Nat. Prod.,* 1989, vol. 52, 1183-1185
- **CONSTANTINO, M. G. ; BELTRAME JR. M. ; DA SILVA, G. V.** *J. Syn. Comm.,* 1996, vol. 26, 321-329
- **LANGE, J.-P. ; BREED, A. J. M.** *Catal. Comm.,* 2002, vol. 3, 25-28
- **OLAH, G. A. ; PARKER, D. G. ; YONEDA, N.** *Angew. Chem. Int. Ed. Engl.,* 1978, vol. 17, 909-931
- **CHEN, B.-C. ; ZHOU, P. ; DAVIS, F. A. ; CIGANEK, E.** *Organic Reactions,* 2004, vol. 64, 1-356
- **YADAV, J. S. ; SATHEESH, B. ; SABITHA, G.** *Tetrahedron Lett.,* 2003, vol. 44, 387-389